# EUROPEAN PATENT APPLICATION

(11) **EP 1 312 920 A1**
(43) Date of publication of application: **21.05.2003**
(21) Application number: 01956879.9
(22) Date of filing: 13.08.2001
(51) Int. Cl.: G01N 33/50, G01N 33/15, G01N 33/566, C12N 15/09

(54) **METHOD OF SCREENING REMEDY FOR HEART FAILURE**

(30) Priority: 22.08.2000 JP 2000250441
(71) Applicant: Yamaguchi Technology Licensing Organization Ltd., Ube-shi, Yamaguchi 755-0039 (JP); Takeda Chemical Industries, Ltd., Osaka-shi, Osaka 541-0045 (JP)
(72) Inventor: UNUI, Makoto, Faculty of Medicine, Ube-shi, Yamaguchi 755-0000 (JP); KIMURA, Yoshihiro, Faculty of Medicine, Ube-shi, Yamaguchi 755-0000 (JP)
(74) Representative: Rickard, Timothy Mark Adrian
(86) International application number: JP0107008
(87) International publication number: WO02016937

(57) **Abstract**

The present invention provides a method for screening a medicine having an action site on a Ca²⁺ transport regulatory mechanism (Ca²⁺ pump - phospholamban system) of the heart muscle endoplasmic reticulum as a Ca²⁺ storage site in the heart muscle cells. In particular, the present invention provides a method for screening a medicine using inhibition of binding between the Ca²⁺ pump and phospholamban.

## Description

### Technical Field

The invention relates to a method for screening medicines for medication of cardiac failures, in particular to a method for screening medicines suitable for medication of chronic cardiac failures.

### Background of Art

Medicines for medication of cardiac failures represented by cardiotonic drugs have been screened using the effects for enhancing cardiac muscle contraction caused by administration to animals or extracted beating hearts as indices. However, the mechanism of pharmacological effect of the medicines are not clarified by these methods, and a vast amount of additional experiments are needed for elucidating possibilities as medicines for medication of cardiac failures. In addition, a lot of animals are needed in these methods with a vast amount of labor and costs.

It has been shown that intracellular Ca²⁺ of the myocardial cells plays a central role as a second messenger in controlling the contraction force of the heart. Intracellular Ca²⁺ of the myocardial cells is mainly controlled by transfer of Ca²⁺ to and release of Ca²⁺ from the myocardial endoplasmic reticulum as a storage site of intracellular Ca²⁺.

The cardiac muscle is relaxed by the decrease of the Ca²⁺ concentration in the cytoplasm due to transfer of Ca²⁺ to the myocardial endoplasmic reticulum, while the cardiac muscle is contracted by the increase of the Ca²⁺ concentration due to release of Ca²⁺ from the myocardial endoplasmic reticulum. All the currently available cardiotonic drugs have a function to increase intake of intracellular Ca²⁺ from the outside of the cells. Accordingly, the drugs rather function to worsen serious cardiac failures, since the decrease of the Ca²⁺ concentration in the cytoplasm becomes insufficient during the relaxing period of the cardiac muscle to fail in sufficiently relax the heart muscle.

The only way for preserving the life of patients with serious chronic cardiac failures who are resistant to such medication is transplantation of the heart or use of an artificial heart.

The problems to be solved by the invention considering the situations as described above are 1) to establish a method for screening medicines for medication of cardiac failures in which the action sites of the medicine is clear in a molecular level; 2) to realize ideal conditions as the medicines for medication of cardiac failures, wherein the drugs selected by screening do not increase intake of Ca²⁺ from the outside of the cells, or an effect for enhancing the myocardial contraction force as well as an effect for promoting myocardial relaxation is exhibited; and 3) to enable simple screening of many compounds without using a lot of experimental animals.

### Disclosure of Invention

For attaining the objects above, the inventors have selected, as an action site of the medicine, a Ca²⁺ transfer regulatory mechanism (a Ca²⁺ pump-phospholamban system) of the myocardial endoplasmic reticulum as a storage site of intracellular Ca²⁺.

The Ca²⁺ pump (Ca²⁺ - ATPase or SERCA2a) in the membrane of the myocardial endoplasmic reticulum is responsible for Ca²⁺ transfer to the myocardial endoplasmic reticulum, and this Ca²⁺ pump further comprises a regulatory mechanism by a membrane protein phospholamban.

It was shown, through intensive studies by the inventors, that phospholamban usually suppresses the Ca²⁺ pump activity by binding to the Ca²⁺ pump, and activates the Ca²⁺ pump by dissociating from the Ca²⁺ pump when necessary.

Relaxation of the heart muscle is accelerated by the increased amount of Ca ²⁺ transfer to the myocardial endoplasmic reticulum when the Ca²⁺ pump of the myocardial endoplasmic reticulum is activated with a simultaneous increase of the heart muscle contraction force due to an increased amount of Ca²⁺ release from the myocardial endoplasmic reticulum. Accordingly, a medicine having an action site on the Ca²⁺ pump - phospholamban system with a relief of suppression of the Ca²⁺ pump activity may be a potentially ideal medicine that can simultaneously achieve acceleration of relaxation and enhancement of contraction of the heart with no increase of Ca²⁺ intake from the outside of the cells.

The present invention also provides a method for screening a medicine that inhibits the binding between the Ca²⁺ pump and phospholamban in order to simply and reliably find a medicine that acts on the action site as has been elucidated as described above.

In a preferred embodiment of the invention, the medicine is screened using a recombinant Ca²⁺ pump protein having a binding site to phospholamban and a recombinant phospholamban protein having a binding site to the Ca²⁺ pump, wherein inhibition of binding between the two recombinant proteins is used as an index for screening. A lot of medicines may be screened using a 96 well micro-plate.

The present invention also provides an ideal medicine for medication of the cardiac failures that is able to specifically eliminate the suppression action of phospholamban to the Ca²⁺ pump while enabling relaxation of the heart to be accelerated and contraction of the heart to be enhanced with no intake of Ca²⁺ from the outside of the cells.

Such a medicine can be efficiently and simply identified by the screening method as described above.

### Brief Description of the Drawings

Fig. 1 is a schematic diagram of the structures of two recombinant proteins used in the example of the screening method according to the invention.
Fig. 2 is a schematic drawing showing the example of the screening method according to the invention.

### Explanation of References

SERCA: a fused protein containing the binding site of Ca²⁺ pump to phospholamban

PLN: a fused protein containing the binding site of phospholamban to Ca²⁺ pump

### Best Mode for Carrying Out the Invention

For developing a screening method of a medicine for medication of cardiac failures according to the present invention, the inventors have elucidated that phospholamban is a regulator protein for the Ca²⁺ pump activity and acts as a suppresser (for example, Inui, M. et al., J. Biol. Chem. 261: 1794-1800, 1986), and that suppression of the Ca²⁺ pump by phospholamban is caused by direct binding between the two proteins, and activation of the Ca²⁺ pump is accelerated by release of phospholamban from the Ca²⁺ pump (for example, Inui, M. et al., J. Biol. Chem. 261: 1794-1800, 1986; James, P. et al., Nature 342: 90-92, 1989). The binding sites of the two proteins have been also identified (for example, James, P. et al., Nature 342: 90-92, 1989; Sasaki, T. et al., J. Biol, Chem. 267: 1674-1679, 1992).

These discoveries elucidate the following facts.

Suppression of the Ca²⁺ pump is relieved and Ca²⁺ pump activity is enhanced by dissociation of phospholamban from the Ca²⁺ pump Enhanced Ca²⁺ pump activity mainly accelerates the amount of intake of Ca²⁺ into the myocardial endoplasmic reticulum to accelerate relaxation of the heart muscle, followed by an increase of the amount of Ca²⁺ storage in the myocardial endoplasmic reticulum due to the increased amount of Ca²⁺ intake. Accordingly, the amount of release of Ca²⁺ from the myocardial endoplasmic reticulum also increases to enhance the contraction force of the heart muscle.

This means that a medicine that can selectively relieve the suppression action of phospholamban to the Ca²⁺ pump is also able to accelerate relaxation of the heart muscle while enhancing the contraction force of the heart muscle, showing that the medicine is an ideal medicine for medication of the cardiac failures.

For finding a novel medicine for medication of the cardiac failures based on the action mechanism as described above, the screening method according to the invention reconstructs the binding between phospholamban and Ca²⁺ pump in vitro. The objective medicine is screened by investigating inhibition of binding between phospholamban and Ca²⁺ pump in the presence of the compound to be tested. The compound that inhibits the binding is a potential candidate of the medicine for medication of the cardiac failures based on the action mechanism as described above.

The screening method according to the invention enables a novel medicine for medication of the cardiac failures acting by a clear mechanism to be efficiently discovered, wherein the medicine has an action site at a Ca²⁺ transfer regulatory mechanism (a Ca²⁺ -phospholamban system) of the myocardial endoplasmic reticulum as a storage site of intracellular Ca²⁺ of the heart muscle.

According to the screening method of the invention, only the medicine acting on the Ca²⁺ pump - phospholamban system is screened. Since no phospholamban is substantially found in the body except the heart, the medicine obtained by the screening is only effective at the heart, and therefore, has little potential danger of side effects. The screening method of the invention enable such medicines to be efficiently screened.

Safety and effectiveness of medication of the cardiac failures based on such action mechanism may be conjectured from knock-out mice of phospholamban created by genetic engineering, because the knock-out mice of phospholamban have no phospholamban, and the mice are conjectured to be in the same condition as the condition in which the Ca²⁺ pump suppressing action of phospholamban is removed by a medicine.

Practically, it is quite reasonable to expect the medicine to be safe and efficient from the facts that no hypertension and shortening of life are observed irrespective of activation of the Ca²⁺ pump of the myocardial endoplasmic reticulum to its maximum (Luo, W. et al., Circ. Res. 75: 401-409, 1994), and that a mouse born by cross-breeding between a phospholamban knock-out mouse and heart muscle failure model mouse shows no cardiac failures syndromes (Minamisawa, S. et. al., Cell 99: 312-322, 1999).

In an example of the preferred embodiment of the screening method of the invention, a recombinant protein labeled at a Ca²⁺ pump partial peptide having a binding site to phospholamban, and a recombinant protein labeled at a phospholamban partial peptide having a binding site to the Ca²⁺ pump are used. The proteins are prepared by assembling cDNAs into respective expression vectors that are expressed in Escherichia coli, and by purifying by taking advantage of their labels.

Medicines that specifically relieve the suppression action of phospholamban to the Ca²⁺ pump may be screened by measuring the amount of binding of two proteins, since binding between the two fused proteins is inhibited so long as the compound tested inhibits the binding between the Ca²⁺ pump and phospholamban. Using a 96 well micro-plate enables simultaneous screening of a lot of compounds at once using a small amount of the compound.

The most crucial feature of the method using the fused proteins prepared by gene recombination is to enable a highly sensitive and simple screening system to be constructed by preparing the labeled proteins that are easily purified and detected. While the examples of the method include those described below, the screening method of the invention is not restricted thereto, and various variations and modifications are possible.

The medicine, which is identified by the screening method of the invention and is able to specifically relieve the suppression action of phospholamban to the Ca²⁺ pump, can achieve acceleration of relaxation of the heart muscle while enhancing the contraction force of the heart muscle with no intake of Ca²⁺ from the outside of the cells. In addition, the medicine is effective only in the heart with little potential danger of any side effects. Such medicine is quite favorable as the medicine for medication of the cardiac failures.

Preferable embodiments of the screening methods of the invention will be described in detail.

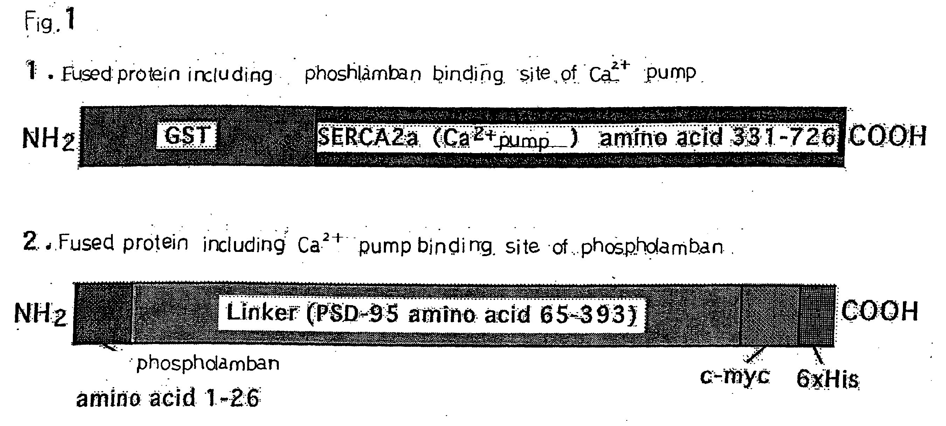

Two recombinant proteins are used in the examples of the invention. The structures of the proteins are shown in Fig. 1.

One is a fused protein (SERCA) in which GST (glutathione S-transferase) is added to a Ca²⁺ pump partial peptide (amino acids No. 331 to 726 of SERCA2a) having a binding site to phospholamban.

The other is a fused protein (PLN) in which c-myc and six histidine residues (6 x His) as a linker and a label, respectively, are added to a phospholamban partial peptide (amino acids No. 1 to No. 26 of phospholamban) having a binding site to the Ca²⁺ pump. Amino acids No. 65 to 393 of PSD-95 protein is used as the PLN linker for facilitating detection in electrophoresis for purification, and for detection with antibodies.

These fused proteins can be readily prepared by assembling corresponding cDNAs into respective expression vectors (pGEX-3X or pTrcHis2) to express in Escherichia coli. SERCA can be purified using a glutathione-sepharose column taking advantage of GST, and PLN can be purified using a Ni-NTA column taking advantage of 6 x His.

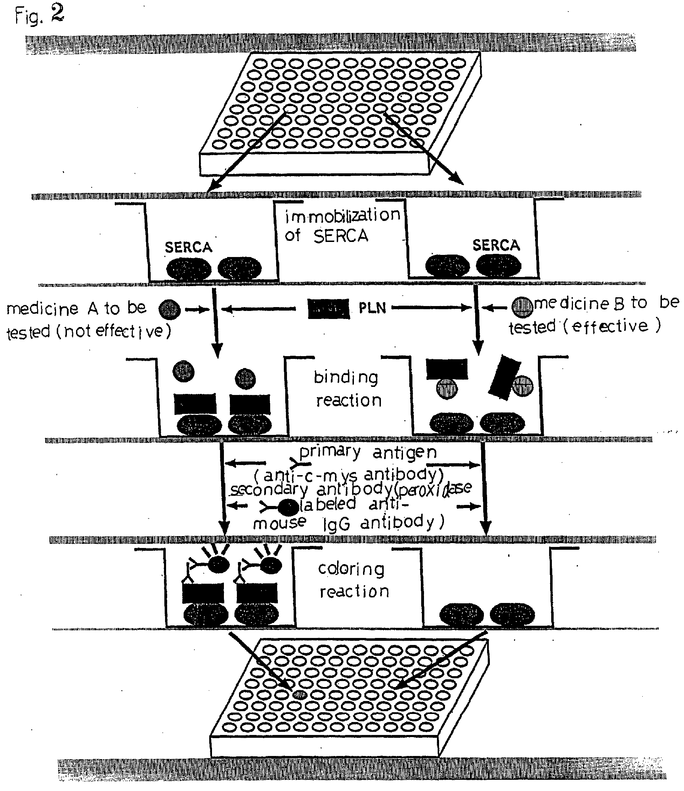

The assay method using the fused protein will be described with reference to Fig. 2.

SERCA is immobilized on each well of the 96 well micro-plate, and the surface of the well is blocked with bovine serum albumin.

Then, a reaction solution containing PLN and a compound to be tested (100 mM KCl, 0.1% Triton X-100, 2 mM 2-mercaptoethanol, 0.5% bovine serum albumin and 20 mM HEPES-HCl, pH 7.5) is added to each well, and the mixture is incubated at room temperature for 2 hours. After washing each well with the same reaction solution, the amount of PLN bound to the immobilized SERCA is detected and quantified using a c-myc antibody or an antibody against the linker as a primary antibody. For detection and quantification, an anti-mouse IgG antibody labeled with peroxidase as a secondary antibody is used, because the c-myc antibody is a mouse monoclonal antibody, and an anti-rabbit IgG antibody labeled with peroxidase as a secondary antibody is used, because the anti-linker antibody is a rabbit antibody. The reaction system is colored by adding tetramethylbenzidine as a coloring substrate, and absorbance at 650 nm is measured using a micro-plate reader.

The intensity of the emitted color is proportional to the amount of binding of PLN to SERCA, which is quantified by measuring absorbance.

As shown by the illustration at the right side in Fig. 2, medicine B is shown to inhibit the binding of PLN to SERCA when the absorbance is decreased by the addition of medicine B, and medicine B is identified to be a medicine that inhibits binding of phospholamban to the Ca²⁺ pump. While an example using the c-myc antibody is shown in Fig. 2, screening using the antigen against the linker is also possible by the same principle as described above.

The function of the screening method according to the invention can be confirmed by using a PLN phosphorylated with a cAMP dependent phosphorylation enzyme or an anti-phospholamban monoclonal antibody called A1 antibody in order to show effectiveness of the method. The studies by the inventors have shown that phospholamban is dissociated from the Ca²⁺ pump by phosphorylation of phospholamban with the cAMP dependent phosphorylation enzyme, and that the A1 antibody inhibits binding between the Ca²⁺ pump and phospholamban.

A remarkable decrease of absorbance or an inhibition of binding of PLN to SERCA is observed when phosphorylated PLN is used in place of PLN, or when the A1 antibody is used in place of the compound to be tested, in the screening method according to the invention. Although the A1 antibody itself has no possibility for serving as a medicine for medication of the cardiac failures, a medicine having the same action as the A1 antibody may be found using the screening method of the invention.

As has been described in detail above, the invention enables whether the medicine to be tested inhibits the binding between the Ca²⁺ pump and phospholamban using the amount of binding between the two fused proteins as an index to be identified, thereby enabling effective screening of the medicine that can specifically relieve the suppressing action of phospholamban to the Ca ²⁺ pump.

This means that acceleration of relaxation as well as enhancement of the contraction force of the heart muscle can be attained with no intake of Ca²⁺ from the outside of the cells. Consequently, an ideal medicine for mediation of the cardiac failures that is effective only in the heart while having little potential danger of side effects may be screened.

While examples of the invention have been described in detail, it is evident that various changes of the features and details of the invention are possible without departing from the scope of the invention defined by claims.

For example, any GST that is attached to the Ca²⁺ partial peptide including the binding site to phospholamban as a label, any the linker attached to the phospholamban partial peptide including the binding site to the Ca²⁺ pump, and any c-myc and 6 x His that are attached as the labels may be used so long as they are effective for purification and detection, and they do not restrict the invention in any sense.

### Industrial Applicability

An ideal medicine for medication of the cardiac failures can be simply and effectively screened by the invention, whereby acceleration of relaxation as well as enhancement of the contraction force of the heart muscle can be attained without increasing the amount of intake of Ca²⁺ from the outside of the cells, and the medicine is effective only in the heart.

Such medicine provides a novel therapeutic means for serious and chronic cardiac failures patients who are difficult for medication, rendering the medicine to be quite useful in medical industries.

## Claims

1. A method for screening a medicine having an action site on a Ca²⁺ transport ragulatory mechanism (Ca²⁺ pump - phospholamban system) of the heart muscle endoplasmic reticulum as a Ca²⁺ storage site in the heart muscle cells.

2. The screening method according to Claim 1 performed by using the amount of binding of the Ca²⁺ pump with phospholamban as an index.

3. The screening method according to Claim 1 or 2 performed by evaluating inhibition of binding between the Ca²⁺ pump and phospholamban in the presence of a compound to be tested.

4. The screening method according to any one of Claims 1, 2 and 3 performed by using a recombinant Ca²⁺ pump protein having a binding site to phospholamban and a recombinant phospholamban protein having a binding site to the Ca²⁺ pump.

5. The screening method according to Claim 4, wherein the recombinant Ca²⁺ pump protein comprises a Ca²⁺ pump partial peptide having a binding site to phospholamban.

6. The screening method according to Claim 5, wherein the recombinant Ca²⁺ pump protein is a recombinant protein labeled at the Ca²⁺ pump partial peptide having a binding site with phospholamban.

7. The screening method according to Claim 5, wherein the recombinant Ca²⁺ pump protein is a fused protein comprising the Ca²⁺ pump partial peptide having a binding site to phospholamban and glutathione S-transferase.

8. The screening method according to Claim 7 wherein the glutathione S-transferase is located at the N-terminal.

9. The screening method according to any one of Claims 5, 6, 7 and 8, wherein the Ca²⁺ pump partial peptide is a peptide comprising amino acids 331 to 726 in the Ca²⁺ pump.

10. The screening method according to Claim 4, wherein the recombinant phospholamban protein comprises a phospholamban partial peptide having a binding site to the Ca²⁺ pump.

11. The screening method according to Claim 10, wherein the recombinant phospholamban protein is a recombinant protein labeled at the phospholamban partial peptide including a binding site to the Ca²⁺ pump.

12. The screening method according to Claim 10, wherein the recombinant phospholamban protein is a fused protein comprising the phospholamban partial peptide including the binding site to the Ca²⁺ pump and c-myc.

13. The screening method according to Claim 10, wherein the recombinant phospholamban protein is a fused protein comprising the phospholamban partial peptide including the binding site to the Ca²⁺ pump, c-myc and six histidine residues.

14. The screening method according to Claim 10, wherein the recombinant phospholamban protein is a fused protein comprising the phospholamban partial peptide including the binding site to the Ca²⁺ pump, a peptide comprising amino acids No. 65 to 393 of a PSD-95 protein, c-myc and six histidine residues.

15. The screening method according to Claim 14, wherein the phospholamban partial peptide is located at the N-terminal, the peptide comprising amino acids No. 65 to 393 of a PSD-95 protein is located at the C-terminal of the phospholamban partial peptide, c-myc is located at the C-terminal of the peptide comprising amino acids No. 65 to 393 of a PSD-95 protein, and six histidine residues is located at the C-terminal of c-myc.

16. The screening method according to any one of Claims 10, 11, 12. 13, 14 and 15, wherein the phospholamban partial peptide is a peptide comprising amino acids 1 to 26 of phospholamban.

17. The screening method according to Claim 1 using the amount of binding of a peptide comprising amino acids No. 331 to 726 of the Ca²⁺ pump with a peptide comprising amino acids No. 1 to 26 of phospholamban as an index.

18. The screening method according to any one of Claims 1, 2, 3 and 4 using a 96 well micro-plate for simultaneously screening a plurality of compounds using a small amount of the compound.

19. The medicine for treating cardiac failures comprising a compound screened by the method according to any one of Claims 1, 2, 3 and 4.

20. The medicine according to Claim 19, wherein the medicine has an accelerating activity for heart relaxation and an enhancing activity for the heart contraction force without any intake of Ca²⁺ from the outside of the cell.
